# EUROPEAN PATENT SPECIFICATION

(11) **EP 2 151 491 B1**
(45) Date of publication and mention of the grant of the patent: **28.10.2015**
(21) Application number: 09009863.3
(22) Date of filing: 30.07.2009
(51) Int. Cl.: C12M 3/00, B01L 3/00

(54) **Multichamber bioreactor with bidirectional perfusion integrated in a culture system for tissue engineering strategies**
Mehrkammer-Bioreaktor mit bidirektionaler Infusion, integriert in einem Kultursystem, zum Einsatz bei Strategien der Gewebetechnik
Bioreacterur multi-chambre avec perfusion bidirectionnel integre dans un systeme de culture pour des strategies d'ingenierie de tissus

(30) Priority: 06.08.2008 PT 10415508
(43) Date of publication of application: 10.02.2010
(73) Proprietor: Associacion for the Advancement of Tissue Engineering and Cell Based Technologies & Therapies (A4TEC), 4710-057 Braga (PT)
(72) Inventor: Costa, Pedro Ferreira da, 4815-495 Caldas de Vizela (PT); Martins, Albino Manuel Pereira, 4820-005 ANTIME Fafe (PT); Gomes, Maria Manuela Estima, Remalde - 4200 Porto (PT); Neves, Nuno João Meleiro Alves das, 4700-207 Braga (PT); Reis, Rui Luís Gonçalves dos, 4250-242 Porto (PT)
(74) Representative: Magalhães Simões, José Raúl

(56) References cited:
- WO-A2-2006/033935
- US-A1- 2002 146 816
- US-A1- 2005 260 745
- HUTMACHER ET AL: "Computational fluid dynamics for improved bioreactor design and 3D culture", TRENDS IN BIOTECHNOLOGY, ELSEVIER PUBLICATIONS, CAMBRIDGE, GB, vol. 26, no. 4, 7 February 2008 (2008-02-07), pages 166-172, XP022537357, ISSN: 0167-7799, DOI: 10.1016/J.TIBTECH.2007.11.012

## Description

### FIELD OF THE INVENTION

The present invention is related to two- and three-dimensional seeding and culture of cells. More particularly, the present invention is related to a bioreactor designed for supporting the seeding and cultivation of cells in two- or three-dimensional constructs for use as medical implants.

### BACKGROUND OF THE INVENTION

Cells in tissues constantly experience mechanical stimuli. Mechanical stress is an important modulator of cell physiology and it is believed that the intracellular mechanical environment is important in tissue homeostasis. Extensive research on the: effect of mechanical stimuli on cell metabolism has suggested that tissues may respond to mechanical stimulation via loading-induced flow of the interstitial fluids. Physical stimuli such as shear-stress, fluid-flow, compression and tension become transduced into biochemical inputs that modulate cellular function. Even cells in static culture experience the effects of gravity. There is considerable evidence that physical stimuli affect gene expression and significantly increase the biosynthetic activity in a range of different cell types. Recent studies have postulated that culturing cells in the appropriate biochemical environment and in the presence of the mechanical stimuli could provide the correct signals for cellular differentiation and production of the desired extracellular matrix (ECM) with appropriate physical properties.

There is considerable interest in developing the appropriate physical environments *in vitro*, using biomechanically active simulation systems, known as bioreactors, to generate or regenerate functional tissues *in vitro*. Bioreactors can be designed in order to establish spatially uniform cell distributions on three dimensional (3D) scaffolds, maintain desired concentrations of gases and nutrients in the culture medium, diffusion of biochemical factors to the construct interior, as well as continuous removal of by-products of cellular metabolism, and to expose developing tissue to appropriate physical stimuli. Such an approach to tissue engineering has the potential to provide not only an essentially unlimited pool of transplants, but also better means to quantitatively control the cell culturing parameters that lead to appropriate tissue development *in vitro.*

Over the past few years several dynamic seeding and culturing systems have been employed to induce different types of physical stimulation to cells *in vitro*. Simple systems include simple dishes, spinner flasks and rotating vessels in which tissue matrices are fixed or floating and the culture medium is exchanged periodically. Other designs are based on perfused columns or chambers in which the tissue matrices are fixed and there is continuous medium recirculation. In these systems, physical conditioning of the tissue-engineered constructs relies upon hydrodynamic shear forces. Providing three-dimensional tissues with nutrients may rely on passive diffusion, or may be more actively delivered by direct perfusion. However, direct perfusion introduces a new level of complexity when scale-up is encountered, and the engineering challenges may be significant.

There are already some documents describing multichamber bioreactors. Patent US 2002/0146816 refers to a single chamber cylindrical biocreactor able to rotate around one of its axis, being medium added and removed from culture during rotation. Patent WO 2006/033935 discloses a bioreactor consisting of a plate with multiple culture chambers which is integrated into a perfusion system. In patent US 2005/260745 a multi-chamber bioreactor within a perfusion system is disclosed.

Moreover, patents WO 2006/033935 and US 2005/260745 consider that each culture chamber has its own culture circuit, which means that the cells within each culture chamber, although being from a same origin, will be cultured independently. As an example, a cell located into one sample can release a substance to the culture medium. In the case of patents WO 2006/033935 and US 2005/260745 that substance will be restricted to and influence other cells within the same sample. In the case of the present invention, since the circulated culture medium is common to all samples, the released substance will act over all cell within all samples.

Summing up, the bioreactors described in patents WO 2006/033935 and US 2005/260745 are focused on independent and isolated cultures of multiple samples while the present invention is focused on the mass production of similar samples in a reproducible way.

In the present application, and unlike patents WO 2006/033935 and US 2005/260745, a single common use peristaltic pump is externally employed. This solution is more economic since peristaltic pumps are common equipments and can be easily adapted to the culture system in multiple configurations.

The present invention aims the development of an enhanced system for seeding and culture of constructs for tissue regeneration, based on the flow perfusion concept. This device is machined in autoclavable and biologically inert materials. The number of culture/perfusion chambers increases significantly the homogeneity between constructs. Its design allows change of the direction and intensity of culture medium flow. Additionally, control over temperature and gas exchange is also achieved through real-time monitoring and automated computer aid. Real-time quantification of cell activity is also possible to perform using appropriate sensors and analytical software.

### DISCLOSURE OF THE INVENTION

The present invention relates to a bioreactor apparatus integrated in a culture system, which may be used for seeding and culturing constructs to be used in tissue regeneration strategies. This bioreactor system may be able to originate constructs for several kinds of tissue implants, such as tissue-engineered bone, skin, cartilage or tendon.

The described bioreactor system is capable of providing continuous perfusion of expansion or differentiation medium, improving the mass transfer rates of cells in the constructs. The continuous perfusion of culture medium provided by the bioreactor system may act as a stimulus to cells, being thus capable of regulating or up-regulating cellular proliferation and differentiation. The perfusion of medium through the cultured constructs can be done either uni- or bi-directionally.

The bioreactor apparatus, which possesses a cylindrical shape, is mainly composed of two lids and a central part, each one of the lids being attachable to each side of the central part. The central part contains several individual culture/perfusion chambers (twenty preferentially), each one capable of retaining one or more scaffolds. When assembled together with the central part, each lid defines a distributor/collector chamber responsible for homogenously feeding culture medium through several constructs. Another main feature of the culture apparatus is its mechanism for retention of constructs inside the culture/perfusion chambers. The constructs are retained in the culture/perfusion chambers, in the centra in between the two distributor/collector chambers. The constructs are kept immobilized inside the culture/perfusion chambers by means of tubular sections whose lengths can be defined according to the thickness of the constructs. Moreover, it is possible to culture either two- or three-dimensional constructs.

The culture/perfusion medium is provided to the distributor/collector chambers through an inlet/outlet port situated in each lid and circulated from and to an aerated medium reservoir by means of a peristaltic pump.

The aeration of the culture medium is performed in the medium reservoir by injection of a pre-established mixture of gases. The gas exchange between the gaseous and the liquid phase may be performed by simple bubbling of the gaseous mixture into the medium, resting in the medium reservoir, or by membrane-through exchange. The mixing of the gases to be injected is performed by a gas mixer, which may be controlled by a central computer. The gases and their concentrations in the medium reservoir may be set according to the type and purpose of each culture.

The temperature inside the culture system is regulated by an integrated electrical thermostatical system connected to and controlled by the central computer. An airtight enclosure is used to contain the core elements of the system in order to maintain an inner constant temperature and to keep a sterile environment.

All the core components of the bioreactor system can be made of inert, biocompatible and autoclavable materials, allowing for an easy and quick sterilization process.

The described bioreactor can be instrumented with sensors for diagnostic measurements of parameters such as hydrostatic pressure., pH, dissolved oxygen (O₂), dissolved carbon dioxide (CO₂) and glucose concentration. Additional analytical systems can be added to the system for quantification of various biological compounds (i. e., metabolites) dissolved into the medium.

Parameters such as medium flow rate and flow sense can also be automated and controlled by connecting the peristaltic pump to the central computer. In this way, these parameters can be maintained according to pre-established culture programs or automatically altered at any time. Medium flow intensity and particularly medium flow bi-directionality are important factors when seeding or culturing cells in a dynamic environment are considered. In this way, our culture system design allows the homogeneity and precise control over these two phenomena.

### BRIEF DESCRIPTION OF THE FIGURES

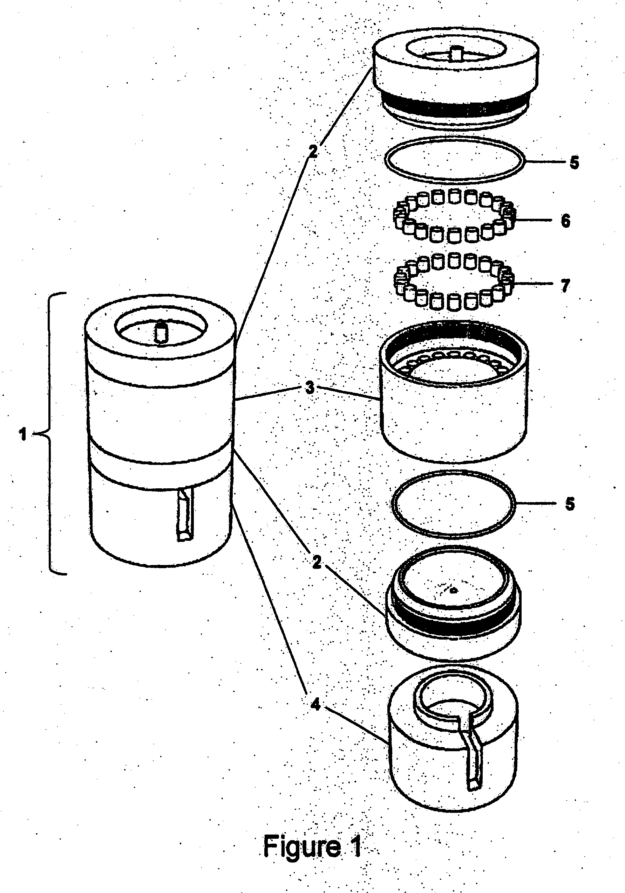

Figure 1 illustrates, in isometric view, the preferred embodiment of the bioreactor apparatus in an exploded view and in an assembled view.

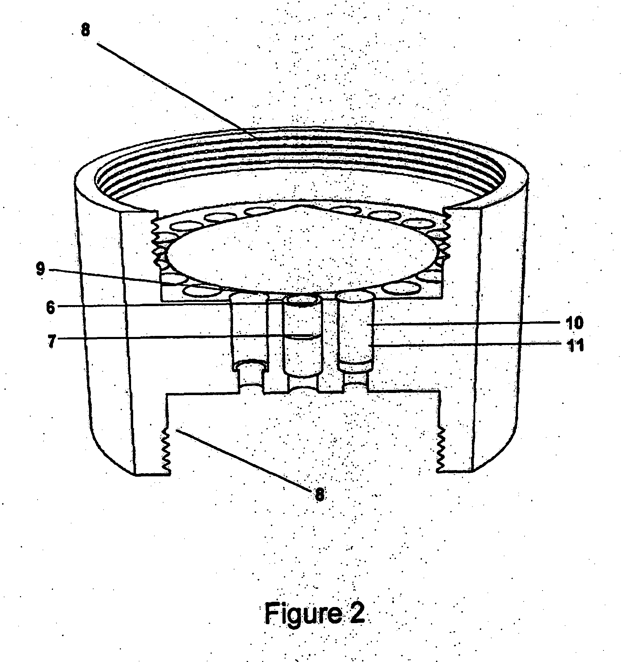

Figure 2 illustrates the central part of the bioreactor apparatus, partially sectioned, showing in detail three of the twenty culture/perfusion chambers in three possible configurations.

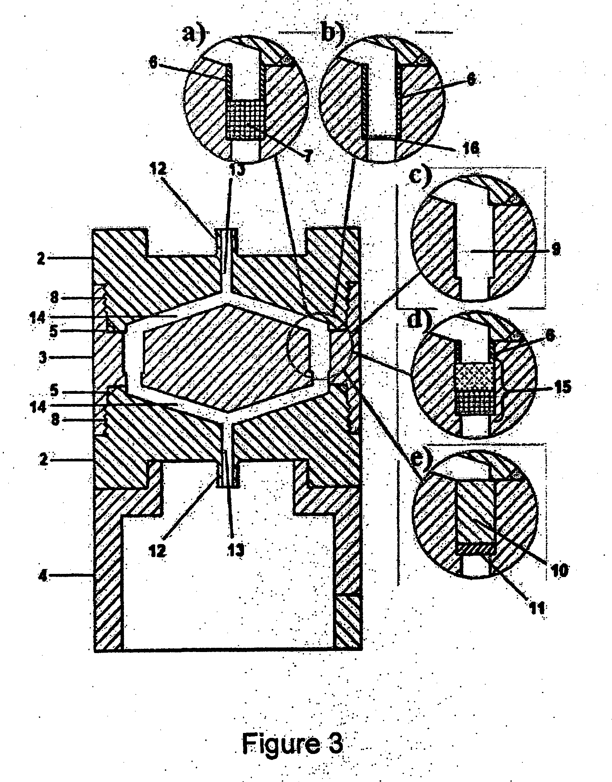

Figure 3 illustrates a longitudinal cross-sectional view of the assembled bioreactor apparatus depicted in Figure 1, showing in detail the culture chambers in five possible configurations: **a)** containing a three-dimensional scaffold sandwiched by a tube section, **b)** containing a membrane sandwiched by a long tube section, **c)** open, **d)** containing two stacked scaffolds sandwiched by a short tube section and **e)** capped by a silicone disc sandwiched by a PTFE cylinder.
Figure 4 illustrates the complete bioreactor system with integrated devices for monitoring and control of medium and environmental composition and medium renewal.

### DETAILED DESCRIPTION OF THE PREFERRED EMBODIMENT

A better understanding of the present invention will be had upon reference to the following description in conjunction with the accompanying drawings.

The bioreactor apparatus (1) is mainly composed of a central part (3), which comprises several culture/perfusion chambers (9) for construct (7) enclosure and two lids (2) that are attached to the central part. Those lids delineate two distributor/collector chambers (14) for culture/perfusion medium that enters/leaves those same distributor/collector chambers through an inlet/outlet port (13) in the centre of each lid.

The central part (3) of this device consists of twenty culture/perfusion chambers, radially disposed, machined into one single cylindrical block of polycarbonate. The use of polycarbonate for building this part relates to its chemical, mechanical and optical properties since it is biologically inert, extremely resistant to solvents and possesses high dimensional stability and resistance to high temperatures. Additionally, this material offers the advantage of being transparent, allowing the contents of each flow chamber to be visualized through the block. Several other materials could be used instead of polycarbonate for the production of this part, especially if the device is intended for discardable purposes. As for the culture/perfusion chambers, their number and dimensions can be varied according to the specific needs of each application. In the invention here described are used twenty chambers with a diameter of eight millimetres and a length of fifteen millimetres, each one capable of containing one or more constructs with eight millimetre diameter and a total thickness of up to ten millimetres.

The constructs are tightly inserted into these culture/perfusion chambers, in order to force the medium to flow through the constructs and not around them. Fluorinated ethylene propylene (FEP) tubular sections (6) are inserted on top of each construct, in order to keep it immobilized inside the culture/perfusion chambers in a constant position. The use of these FEP tubular sections is an important and significant feature of the invention since it allows to culture constructs of different heights by simply adjusting the length of the tubular sections which can be cut-off from eight millimeter outer diameter FEP transparent tubes. As for the inner diameter of the FEP tubular sections, it should be as large as possible but small enough to tightly hold the constructs in place. Like all other parts, other materials may be considered for obtaining these tubular sections as long as they possess adequate characteristics.

As samples are removed from the culture/perfusion chambers at different time points, each remaining cavity is capped with a silicone disc (11) and a polytetrafluoroethylene (PTFE) cylinder (10) in order to completely stop the perfusion medium from going through the empty chambers. After removal of constructs, the total flow perfusion rate is re-set in order to maintain the desired flow rate being perfused across each individual construct.

Due to the versatility of the designed culture/perfusion chambers and construct retention mechanism, multiple scaffold/construct configurations can be used. Apart from single three-dimensional scaffolds/constructs (7), each culture/perfusion chamber can also contain multiple scaffold/constructs, such as multiple stacked scaffolds/constructs (15). Additionally, by adjusting the length of the FEP tubular sections (6), two-dimensional scaffolds or membranes (16) can also be used.

In order to close the apparatus, the two lids (2) made of PTFE are attached to the central part. This attachment is achieved through the use of a screw mechanism enabled by the presence of screw threads (8) in both lids and in the central part. The choice of using PTFE for the fabrication of the two lids is related to its excellent dimensional stability, high resistance to temperature, constant mechanical properties and for being an inert and biocompatible material. In order to guarantee the capping of the gaps between the lids and the central polycarbonate part, two Viton^{®} o-rings (5) are used.

Each one of the two lids delineates a distributor/collector chamber (14) which can act both as a medium distributor through the several culture/perfusion chambers and as a medium collector, depending on the selected sense of the perfusion medium. The surfaces of these chambers were designed in a way that the medium being circulated through them is equally distributed between the several flow chambers. In this way, all the constructs inserted in the culture/perfusion chambers are exposed to equal flow conditions. The main advantage of this medium-distributing system is that it is integrated in the culture apparatus. This feature eliminates the need for using multiple tubes and tube connections, which are greatly responsible for increasing the risk of contamination. Additionally, this design minimizes the possibility of air bubble accumulation. Also, it makes the system considerably simple, user friendly and, consequently, allows a very easy and fast assembling.

Each one of the lids (2) feature a central port (13) that connects the perfusion medium chambers to a screw threaded tube (12) that allows the connection of outer tubes for medium circulation. The design of the lids also allows for the bioreactor apparatus to fit on a specially designed cradle (4) made of PTFE that supports the apparatus during assembly and culture.

Since both lids are exactly the same, both lids are able to attach to both sides of the central part and to fit in the support cradle as it is also possible to quickly and easily invert the bioreactor apparatus at any time.

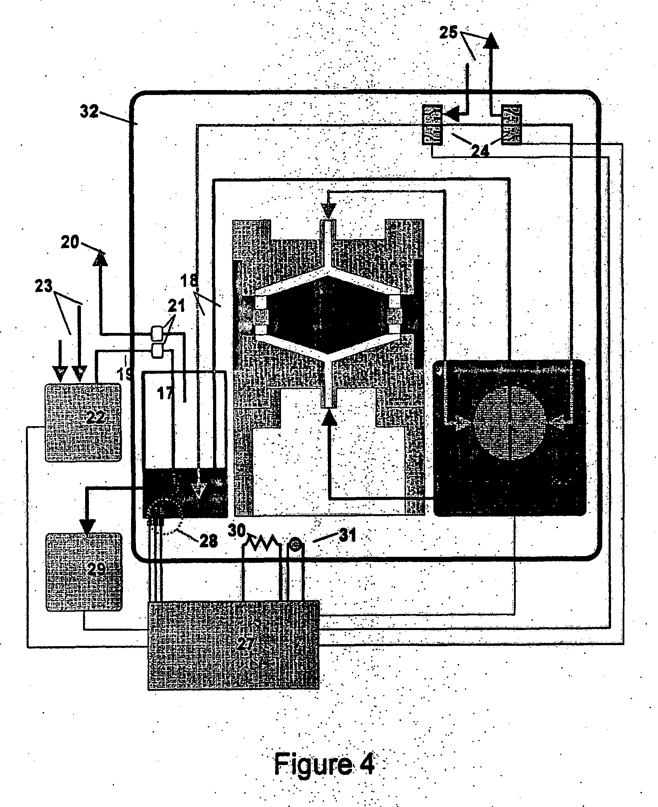

As for the bioreactor system illustrated in Fig. 4, it is composed of a bioreactor apparatus (1) (previously described) connected by both screw threaded tubes (12) in both ends of the assembled bioreactor apparatus to a common medium reservoir (17) which, apart from two medium inlet/outlet ports (18), contains extra ports for gas inlet (19) and outlet (20) capped by air filters (21). The inlet gas filter can be directly exposed to surrounding atmospheric gas mixture (option not shown) or can alternatively and preferably be connected to a gas mixer (22) which continuously mixes and injects a desired mixture of various gases obtained from external sources (23) through the air filter into the culture medium contained in the medium reservoir (17). In this way it is possible to control the concentration of each gas dissolved into the circulating culture medium. The gases are dissolved into the culture medium by simple bubbling of the gaseous mixture into the culture medium or alternatively through a gas-permeable membrane (not shown) immersed into the culture medium.

In brief, the medium is collected from the common medium reservoir (17), pumped into one medium distributor/collector chamber (14), perfused through the constructs contained in the multiple culture/perfusion chambers, collected in a second medium distributor/collector chamber (14) and redirected back into the common medium reservoir (17). Additionally, computer controlled pinch valves (24) combined with medium inlets/outlets (25) can be inserted into the' medium flow circuit in order to automatically discard the circulating culture medium and simultaneously insert fresh one coming from an external source/reservoir.

The tubing used for circulating the perfusing medium can be of various kinds. When a gas mixer is used for dissolving gases into the culture medium it is preferred that the tube used has low or no permeability to gases in order to maintain a constant and precise gaseous composition dissolved into the circulating medium. Tubing made of formulations such as Tygon^{®} R-3607 or Pharmed^{®} ismaprene are good examples. When gases are passively dissolved into the circulating medium simply using a filter capping, it is preferred that the tubing used is permeable to gases in order to increase the exchange of gases between the circulating medium and the outer atmosphere. In this case, formulations such as platinum-cured silicone could be used. This kind of tubing is highly gas permeable to both carbon dioxide and oxygen.

One of the most important features of this bioreactor is that, due to the culture chamber design and the flow circuit arrangement, it is possible to invert the flow direction and to change the flow rate of the perfusing medium at any time by simply changing the and speed of the peristaltic pump (26) in the perfusion circuit, allowing to perfuse medium through scaffolds in a bi-directional way with a desired intensity. Thereby, it is possible to easily apply perfusion programs that invert the flow direction and change the intensity of the medium flow as desired along the duration of the seeding/culture.

The establishment of flow intensity and flow sense programs can be implemented both manually, by changing the flow speed and sense in the peristaltic pump display at desired time points, or automatically, by using a computer (27) to control the peristaltic pump's rotation speed and sense. When seeding cells into scaffolds, the establishment of an adequate medium flow intensity is crucial for an effective adhesion of cells to the surfaces of the scaffolds while the bi-directionality is responsible for a more homogenous distribution of cells over the whole scaffold's inner and outer surfaces. During culture, the application of an adequate medium flow rate combined with a suitable program for inversion of the medium sense can be responsible for enhanced proliferation and differentiation according to the chosen culture program.

This system also allows, if desired, for the connection of several sensors (28) in order to monitor parameters such as hydrostatic pressure, pH, dissolved O₂, dissolved CO₂, and glucose concentration, among others. Additionally, analytical systems (29) can be added to the system. Systems based on analytical technologies such as flow injection analysis (FIA) or sequential injection analysis (SIA) can be integrated into the system in order to periodically collect samples from the circulating perfusion medium and to quantify several biochemical compounds (i. e., cell metabolites) dissolved into the medium. All the data acquired through all these sensors and analyzers, can be acquired independently into each sensor's software or directed to a central software application in the central computer (27) that can be at the same time responsible for the automation and control of all the electronically driven devices in the system, such as the peristaltic pump and valves. This solution would allow for an easy and centralized acquisition of data synchronized with the actions of the effector devices integrated in the bioreactor system.

The temperature inside the culture system is regulated by an integrated electrical resistance (30) and a temperature sensor (31), which are also connected to and controlled by the central computer (27).

In order to maintain a sterile and temperature-stable environment, the core elements of the system are placed into an airtight enclosure (32). This enclosure contains several ports for medium, gases and for data communication. This enclosure can be made of polycarbonate or similar materials that allow the enclosed components of the system to be observed from the outside and also so that the enclosure can be quickly and easily sterilized by autoclaving.

As previously mentioned, this bioreactor system may be suitable not only for culturing but also for the seeding of cells into scaffolds. Due to its small dimensions, this system requires very small quantities of medium to be able to efficiently perfuse scaffolds or constructs. For this reason, it is possible to perform a dynamic seeding procedure using highly concentrated cell suspensions without needing extremely high quantities of cells. Usually, a volume of about 100 ml is enough to perform a dynamic seeding into a set of scaffolds, since the maximum total volume of medium contained in the interior of the perfusion circuit is about 90 ml. In this way, cells have a greater chance to attach to the scaffold's surfaces since they are in greater concentration and pass through the scaffolds for a higher number of times and so, the dynamic seeding can be more effective.

Also, due to the bidirectional nature of this culturing system, the cell seeding becomes more homogenous. The constant change on the direction of the perfusing cell suspension flow allows variations on the conditions to which the multiple surfaces of the scaffold are exposed to. In this way, all the surfaces of the scaffold tend to feel the same varying flow intensity, independently of its position or orientation in the scaffold.

## Claims

1. Bioreactor apparatus (1) **characterized by** being mainly composed of a central part (3), which comprises several culture/perfusion chambers (9) radially disposed, machined into one single cylindrical block of polycarbonate between two distributor/collector chambers (14), for construct (7) enclosure and two lids (2) that are attached to the central part through the use of a screw mechanism enabled by the presence of screw threads (8) in both lids and in the central part; said lids delineate two distributor/collector chambers (14) for culture/perfusion medium that will be equally distributed by the culture/perfusion chambers (9), said medium entering through a single inlet (13) in the center of the distributor chamber lid and exiting through a single outlet (13) in the center of the collector chamber lid allowing for an inter-sample homogenous culture; the central ports (13) connect the perfusion medium chambers to a screw threaded tube (12) that allows the connection of outer tubes for medium circulation; the design of the lids also allows for the bioreactor apparatus to fit on a specially designed cradle (4).

2. Bioreactor according to claim 1 **characterized by** having both lids (2) exactly identical, allowing for a quick and easy inversion of the bioreactor apparatus at any time.

3. Bioreactor according to claims 1 and 2, **characterized in that** the number of culture/perfusion chambers reaching an optimal area to volume ratio is 20.

4. Bioreactor according to claims 1 to 3, **characterized in that** it retains the constructs into the culture/perfusion chambers, in the central part, between the two distributor/collector cavities; the constructs are kept immobilized by using tubular sections whose lengths can be defined according to the thickness of the constructs.

5. Bioreactor, according to claims 1 to 4, **characterized by** having each one of the two lids delineating a distributor/collector chamber (14), which can act both as a medium distributor through the several culture/perfusion chambers and as a medium collector, depending on the selected sense of perfusion medium.

6. Bioreactor, according to claims 1 to 5, **characterized by** having a culture medium distribution mechanism integrated in the bioreactor itself.

7. Bioreactor, according to claims 1 to 6, **characterized by** allowing the culture of two- or three-dimensional constructs.

8. Bioreactor, according to claims 1 to 7, **characterized by** allowing the use of any type of tubing whose dimensions are consistent with the bioreactor in- and out-flow, and made of a biocompatible and inert material.

9. Bioreactor, according to claims 1 to 8, **characterized by** allowing each culture/perfusion chamber to accommodate one or more scaffolds.

10. Bioreactor, according to claims 1 to 8, **characterized by** allowing the main components of the culture system to be constructed from any biocompatible or inert material.

11. Cell culture system **characterized in that** it is composed of a bioreactor (1) according to claim 1, connected to a medium reservoir (17) through inlet/outlet ports (13) coming from both lids which possesses, apart from two medium inlet/outlet ports (18), an additional inlet (19) and outlet (20) for gases which are purified by air filters (21); the medium is collected from the common medium reservoir (17), pumped into one medium distributor/collector chamber (14), perfused through the constructs contained in the multiple culture/perfusion chambers, collected in a second medium distributor/collector chamber (14) and redirected back into the common medium reservoir (17); **in that** the computer controlled pinch valves (24) combined with medium inlets/outlets (25) can be inserted into the medium flow circuit in order to automatically discard the circulating culture medium and simultaneously insert fresh one coming from an external source/reservoir; and **in that** the perfusion of expansion or differentiation medium through the cultured constructs can be done either uni- or bi-directionally.

12. Cell culture system, according to claim 11, **characterized by** allowing the temperature inside the culture system to be regulated by an integrated electrical thermostatical system connected to and controlled by the central computer.

13. Cell culture system, according to the former claims, **characterized by** possessing a medium circuit that includes a unique medium reservoir for collection and distribution of medium from and to the culture/perfusion chambers.

14. Cell culture system, according to the former claims, **characterized by** allowing the aeration of the culture medium to be performed in the medium reservoir by injection (bubbling) of a pre-established mixture of gases, automatically monitored and controlled by a central computer, or by passive filter-through exchange with the external atmosphere.

15. Cell culture system, according to the former claims, capable of being used as a dynamic culturing system, with medium flow established uni- or bi-directionally.

16. Cell culture system, according to the former claims, **characterized by** allowing the empty cavities resulting from the removal of constructs at different time points to be capped with a silicone disc (11) and cylinder (10) made of polytetrafluoroethylene (PTFE) or any other compatible material, in order to completely stop the perfusion medium from going through the empty chambers, the total flow perfusion rate being re-set in order to maintain the desired flow rate being perfused across each individual construct, after removal of constructs.

17. Cell culture system, according to the former claims, **characterized by** allowing the integration of multiple sensors (28) for monitoring parameters such as hydrostatic pressure, pH, dissolved oxygen and carbon dioxide and glucose concentration, among others, as well as other analytical equipments such as those based on flow injection analysis (FIA) or sequential injection analysis (SIA) could be coupled to the bioreactor system, in order to allow evaluating the biochemical components dissolved in the medium and the physical conditions established.

18. Cell culture system, according to the former claims, **characterized by** having its main elements placed inside a sealed chamber (32) containing several holes for medium culture and gases exchange.

19. Cell culture system, according to the former claims, **characterized by** allowing the use of any cell type (i.e., human or animal origin, primary culture or cell line) for seeding and/or culturing procedures.

20. Cell culture system, according to the former claims, **characterized by** allowing for its main components to be fabricated from inert, biocompatible and autoclavable materials, allowing for an easy and quick sterilization process.

## Patentansprüche

1. Bioreaktorvorrichtung (1), die im Wesentlichen **dadurch gekennzeichnet ist, dass** sie aus einem Mittelteil (3) besteht, der mehrere radial angeordnete Kultur-/Perfusionskammern (9) umfasst und in einem einzigen zylindrischen Block aus Polycarbonat zwischen zwei Verteiler-/Kollektorkammern (14) verarbeitet ist, der als Gehäuse (7) dient und mit zwei Gehäusedeckeln (2) ausgestattet ist, die mit dem zentralen Teil durch die Verwendung eines Schraubenmechanismus verbunden sind, der durch das Vorhandensein von Schraubgewinden (8) in den beiden Deckeln und im zentralen Teil funktioniert; die Deckel grenzen zwei Verteiler-/Kollektorkammern (14) für das Nähr-/Perfusionsmedium ab, das gleichermaßen von den Kultur-/Perfusionskammern (9) verteilt wird, wobei das eintretende Medium über einen einzigen Einlass (13) in der Mitte des Verteilerkammerdeckels durch einen einzigen Auslass (13) in der Mitte des Kollektorkammerdeckels fließt, so dass eine homogene Kultur für eine übergreifende Probe möglich ist; die Zentralöffnungen (13) verbinden die Kammern des Perfusionsmediums mit einem mit einem Gewinde versehenen Rohr (12), das den Anschluss von Außenschläuchen für die Zirkulation des Mediums ermöglicht; das Design der Deckel macht es möglich, dass der Bioreaktor auf eine speziell entwickelte Ladestation (4) passt.

2. Der Bioreaktor ist gemäß Anspruch 1 **dadurch gekennzeichnet, dass** die beiden Deckel (2) exakt identisch sind, was zu jeder Zeit eine einfache und schnelle Umkehrung der Bioreaktorvorrichtung ermöglicht.

3. Der Bioreaktor ist gemäß den Ansprüchen 1 und 2 **dadurch gekennzeichnet, dass** die Anzahl der Kultur-/Perfusionskammern ein optimales A/V-Verhältnis von 20 erreicht.

4. Der Bioreaktor ist gemäß den Ansprüchen 1 bis 3 **dadurch gekennzeichnet, dass** es die Konstrukte in den Kultur-/ Perfusionskammern zurückhält, im zentralen Teil zwischen den beiden Verteiler-/Kollektorhohlräumen; die Konstrukte werden durch die Verwendung rohrförmiger Abschnitte, deren Länge entsprechend der Dicke der Konstrukte definiert werden kann, immobilisiert.

5. Der Bioreaktor ist gemäß den Ansprüchen 1 bis 4 **dadurch gekennzeichnet, dass** jeder der beiden Deckel eine Verteiler-/Kollektorkammer (14) abgrenzt, die sowohl als Verteiler des Mediums durch die verschiedenen Kultur-/Perfusionskammern als auch als Kollektor für das Medium dienen kann, je nach der Richtung, in der sich das Medium bewegen soll.

6. Der Bioreaktor ist gemäß den Ansprüchen 1 bis 5 **dadurch gekennzeichnet, dass** der Verteilmechanismus für das Nährmedium in den Bioreaktor selbst eingebaut ist.

7. Der Bioreaktor ist gemäß den Ansprüchen 1 bis 6 **dadurch gekennzeichnet, dass** er die Kultur von zwei- oder dreidimensionalen Konstrukten möglich macht.

8. Der Bioreaktor ist gemäß den Ansprüchen 1 bis 7 **dadurch gekennzeichnet, dass** er die Verwendung von jeder Art von Schläuchen möglich macht, deren Größe mit dem Ein- und Ausfluss des Bioreaktors übereinstimmen und die aus einem biokompatiblen und inertem Material hergestellt sind.

9. Der Bioreaktor ist gemäß den Ansprüchen 1 bis 8 **dadurch gekennzeichnet, dass** in jeder Kultur- /Perfusionskammer ein oder mehrere Gerüste untergebracht werden können.

10. Der Bioreaktor ist gemäß den Ansprüchen 1 bis 8 **dadurch gekennzeichnet, dass** die Hauptbestandteile des Kultursystems aus jedem beliebigen biokompatiblen oder inerten Material gebaut werden können.

11. Das Zellkultursystem ist **dadurch gekennzeichnet, dass** es aus einem Bioreaktor (1) gemäß Anspruch 1 besteht, der durch Einlass-/Auslassöffnungen (13), die durch die beiden Deckel kommen, die er besitzt, mit einem Mediumbehälter (17) verbunden ist, neben zwei weiteren Ein-/Auslassöffnungen (18) für das Medium und einem zusätzlichen Ein- (19) und Auslass (20) für Gase, die durch Luftfilter gereinigt werden (21); Das Medium wird von der gemeinsamen Mediumbehälter (17) gesammelt, in eine Mittelverteiler/-kollektor (14), durch die in den Mehrfachkultur-/Perfusionskammern enthaltenen Konstrukten geströmt, in einer zweiten Verteiler-/Kollektorkammer gesammelt (14) und umgeleitet in den gemeinsamen Mediumbehälter (17), in den die computergesteuerten Quetschventile (24) in Verbindung mit den Ein-/Auslässen für das Medium (25) in den Strömungskreislauf eingefügt werden können, um das zirkulierende Nährmedium automatisch zu verwerfen und gleichzeitig frisches aus einer externen Quelle/Behälter einführen zu können; und dass die Perfusion des Expansions- oder Differenzierungsmediums durch die kultivierten Konstrukte entweder un- oder bidirektional erfolgen können.

12. Das Zellkultursystem ist gemäß Anspruch 11 **dadurch gekennzeichnet, dass** die Temperatur im Inneren des Kultursystem durch ein integriertes elektrisches thermostatisches System reguliert wird, das zur Steuerung mit einem zentralen Computer verbunden ist.

13. Das Zellkultursystem ist gemäß den vorhergehenden Ansprüchen **dadurch gekennzeichnet, dass** es über einen Medium-Kreislauf, zu dem ein einzigartiger Mediumbehälter gehört zur Aufnahme und Verteilung von Medium von und zu den Kultur-/Perfusionskammern.

14. Das Zellkultursystem ist gemäß der vorhergehenden Ansprüche **dadurch gekennzeichnet, dass** die Belüftung des Nährmediums im Behälter des Mediums durch Injektion (Blasenbildung) einer vorher festgelegten Mischung von Gasen durchgeführt wird und von einem zentralen Computer automatisch überwacht und gesteuert wird, oder durch passiven Austausch mit der Außenatmosphäre durch einen Filter.

15. Das Zellkultursystem ist gemäß der vorhergehenden Ansprüche in der Lage ist, als ein dynamisches Kultursystem verwendet zu werden, mit einem bestimmten uni- oder bidirektionalen Fluss des Mediums.

16. Das Zellkultursystem ist gemäß der vorhergehenden Ansprüche **dadurch gekennzeichnet, dass** die leeren Hohlräume, die aus der Entfernung von Konstrukten zu verschiedenen Zeitpunkten resultieren, mit einer Silikonscheibe (11) und Zylinder (10) aus Polytetrafluoräthylen (PTFE) abgedeckt werden können, oder jedem anderen kompatiblen Material, um zu verhindern, dass das Perfusionsmedium durch die leeren Kammern läuft, wobei die gesamte Perfusionsflussrate neu eingestellt wird, damit die gewünschte Durchflussrate, die durch jedes Konstrukt läuft, nach dem Entfernen der Konstrukte beibehalten wird.

17. Das Zellkultursystem ist gemäß der vorhergehenden Ansprüche **dadurch gekennzeichnet, dass** sowohl mehrere Sensoren (28) zur Überwachung von Parametern wie dem hydrostatischen Druck, den pH-Wert, den gelösten Sauerstoff und Kohlendioxid und die Glucose-Konzentration, unter anderem, als auch andere analytische Geräte, wie solche, die auf Fließinjektionsanalyse (FIA) oder sequentielle Injektionsanalyse (SIA) basieren, an das Bioreaktorsystem gekoppelt werden können, damit die in dem Medium gelösten biochemischen Komponenten und die festgelegten physikalischen Bedingungen beurteilt werden können.

18. Das Zellkultursystem ist gemäß den vorher gehenden Ansprüchen **dadurch gekennzeichnet, dass** sich seine Hauptelemente in einer abgedichteten Kammer (32) befinden, mit mehreren Löchern für das Nährmedium und den Gasaustausch.

19. Das Zellkultursystem ist gemäß den vorher gehenden Ansprüchen **dadurch gekennzeichnet, dass** es die Verwendung von jedem Zelltyp (z.B. menschlichen oder tierischen Ursprungs, Primärkulturen oder Zelllinien) für Saat- und/oder Kulturverfahren zulässt.

20. Das Zellkultursystem ist gemäß den vorher gehenden Ansprüchen **dadurch gekennzeichnet, dass** seine Hauptbestandteile aus inerten, biokompatiblen und autoklavierbaren Materialien hergestellt sind, die einen einfachen und schnellen Sterilisationsprozess gewährleisten.

## Revendications

1. Appareil bioréacteur (1) **caractérisé en ce qu'**il est principalement composé d'une partie centrale (3), laquelle comprend plusieurs chambres de culture/perfusion (9) disposées radialement, usinées dans un seul bloc cylindrique de polycarbonate entre deux chambres de distribution/collecte (14), pour enceinte de construit (7) et deux couvercles (2) qui sont reliées à la partie centrale par le biais d'un mécanisme à vis possibilité par la présence de filets de vis (8) dans les deux couvercles et dans la partie centrale; lesdits couvercles délimitent deux chambres de distribution/collecte (14) pour le milieu de culture/perfusion qui seront également distribués par les chambres de culture/perfusion (9), ledit milieu entrant par une seule entrée (13) au centre du couvercle de la chambre de distribution et sortant par une seule sortie (13) au centre du couvercle de la chambre de collecte permettant une culture homogène inter-échantillon; les ports centrales (13) relient les chambres de milieu de perfusion à un tube fileté (12) qui permet la connexion de tubes extérieurs pour la circulation du milieu; la conception des couvercles permet aussi que l'appareil bioréacteur s'ajuste dans un berceau spécialement conçu (4).

2. Bioréacteur selon la revendication 1, **caractérisé en ce qu'**il possède les deux couvercles (2) exactement identiques, ce qui permet une inversion rapide et facile de l'appareil bioréacteur à tout moment.

3. Bioréacteur selon les revendications 1 et 2, **caractérisé en ce que** le nombre de chambres de culture/perfusion qui atteint un rapport optimale surface/volume est 20.

4. Bioréacteur selon l'une des revendications 1 à 3, **caractérisé en ce qu'**il maintient les construits dans les chambres de culture/perfusion, dans la partie centrale, entre les deux cavités de distribution/collecte; les construits sont maintenus immobilisés par l'utilisation de sections tubulaires dont les longueurs peuvent être définis en fonction de l'épaisseur des construits.

5. Bioréacteur selon les revendications 1 à 4, caractérisé en ce qui chacun des deux couvercles délimitent une chambre de distribution/collecte (14), qui peut agir à la fois comme un distributeur de milieu par l'intermédiaire de plusieurs chambres de culture/perfusion et comme un collecteur de milieu, en fonction du sens du milieu de perfusion choisi.

6. Bioréacteur selon les revendications 1 à 5, **caractérisé en ce qu'**il possède un mécanisme de distribution de milieu de culture intégré dans le bioréacteur lui-même.

7. Bioréacteur selon les revendications 1 à 6, **caractérisé en ce qu'**il permet la culture de construits à deux ou trois dimensions.

8. Bioréacteur selon les revendications 1 à 7, **caractérisé en ce qu'**il permet l'utilisation de tout type de tubes dont les dimensions sont compatibles avec l'entrée et la sortie de milieu du bioréacteur et fabriqués d'un matériau soit biocompatible ou inerte.

9. Bioréacteur selon les revendications 1 à 8, **caractérisé en ce qu'**il permet que chaque chambre de culture/perfusion accommode un ou plusieurs supports.

10. Bioréacteur selon les revendications 1 à 8, **caractérisé en ce qu'**il permet que les composants principaux du système de culture soient construits d'un quelconque matériau biocompatible ou inerte.

11. Système de culture cellulaire **caractérisé en ce qu'**il comprend un bioréacteur (1) selon la revendication 1, relié à un réservoir de milieu (17) par l'intermédiaire de ports d'entrée/sortie (13) provenant des deux couvercles qui possèdent, en dehors des deux ports d'entrée/sortie de milieu (18), une entrée (19) et une sortie supplémentaires (20) pour des gaz qui sont purifiés par des filtres à air (21); le milieu est collecté du réservoir commun de milieu (17), pompé dans une chambre de distribution/collecte de milieu (14), perfusé à travers de construits contenues dans les multiples chambres de culture/perfusion, collecté dans une seconde chambre de distribution/collecte de milieu (14) et réorienté vers le réservoir de commun de milieu (17); **caractérisé en ce que** les vannes à manchon commandés par ordinateur (24) combinées avec les entrées/sorties de milieu (25) peuvent être insérées dans le circuit d'écoulement de milieu pour déchirer automatiquement le milieu de culture circulant et simultanément d'insérer un nouveau milieu provenant d'une source/réservoir externe; et **caractérisé en ce que** la perfusion d'expansion ou le milieu de différenciation à travers les construits en culture peut être fait soit uni- ou bidirectionnellement.

12. Système de culture cellulaire selon la revendication 11, **caractérisé en ce qu'**il permet à la température à l'intérieur du système de culture d'être régulée par un système à thermostatique électrique intégré connecté à et contrôlé par l'ordinateur central.

13. Système de culture cellulaire selon l'une quelconque des revendications précédentes, **caractérisé en ce qu'**il possède un circuit de milieu qui comprend un seul réservoir de milieu pour la collecte et la distribution du milieu de et vers les chambres de culture/perfusion.

14. Système de culture cellulaire selon l'une quelconque des revendications précédentes, **caractérisé en ce qu'**il permet l'aération du milieu de culture à effectuer dans le réservoir de fluide par injection (bouillonnement) d'un mélange préétabli de gaz, contrôlé automatiquement et commandé par un ordinateur central, ou par échange de filtre passive avec l'atmosphère extérieure.

15. Système de culture de cellules selon l'une quelconque des revendications précédentes, capable d'être utilisés comme un système de culture dynamique, avec un débit de milieu établi uni- ou bidirectionnelle.

16. Système de culture cellulaire selon l'une quelconque des revendications précédentes, **caractérisé en ce qu'**il permet aux cavités vides résultant de l'élimination de construits à différents points dans le temps d'être bouchées avec un disque de silicone (11) et le cylindre (10) fabriqué en polytetrafluoroethylene (PTFE) ou de tout autre matériau compatible, pour arrêter complètement le milieu de perfusion de passer à travers les chambres vides, le taux de perfusion du débit total étant de nouveau réglé pour maintenir le taux de débit souhaité qui est en trains d'être perfusé à travers chaque construit individuelle, après l'enlèvement des construits.

17. Système de culture cellulaire selon l'une quelconque des revendications précédentes, **caractérisé en ce qu'**il permet l'intégration de multiples capteurs (28) pour des paramètres de surveillance comme la pression hydrostatique, le pH, l'oxygène dissous et le dioxyde de carbone et la concentration de glucose, entre autres, ainsi que d'autres équipements analytiques telles que ceux basés sur l'analyse par injection en flux (FIA) ou une analyse par injection séquentiel (SIA) peuvent être accouplés au système de bioréacteur afin de permettre l'évaluation les composants biochimiques dissous dans le milieu et les conditions physiques établies.

18. Système de culture cellulaire selon l'une quelconque des revendications précédentes, **caractérisé en ce qu'**il possède ses principaux éléments placés à l'intérieur d'une chambre étanche (32) contenant plusieurs trous pour la culture de milieu et échange des gaz.

19. Système de culture cellulaire selon l'une quelconque des revendications précédentes, **caractérisé en ce qu'**il permet l'utilisation de tout type de cellule (par exemple, d'origine humaine ou animale, culture primaire ou une lignée cellulaire) pour l'ensemencement et/ou procédures de culture.

20. Système de culture cellulaire selon l'une quelconque des revendications précédentes, **caractérisé en ce qu'**il permet qui ses composantes principaux soient fabriqués à partir des matériaux inertes, biocompatibles et autoclavables, permettant un processus de stérilisation facile et rapide.
